# EUROPEAN PATENT SPECIFICATION

(11) **EP 0 703 906 B1**
(45) Date of publication and mention of the grant of the patent: **30.08.2000**
(21) Application number: 94919492.2
(22) Date of filing: 13.06.1994
(51) Int. Cl.: C07D 251/26, C07D 251/46, C07D 251/44, C07D 251/52, C09B 62/445, C09B 62/505

(54) **FIBER REACTIVE ANTHRAQUINONE DYES**
FASERREAKTIVE ANTHRACHINON-FARBSTOFFE
COLORANTS ANTHRAQUINONIQUES REACTIFS AUX FIBRES

(30) Priority: 14.06.1993 US 77225
(43) Date of publication of application: 03.04.1996
(73) Proprietor: DyStar L.P., Charlotte, North Carolina 28273 (US)
(72) Inventor: PEDEMONTE, Ronald, P., Coventry, RI 02816 (US); HELMLING, Walter, 65817 Eppstein (DE)
(74) Representative: Muley, Ralf, Dr.
(86) International application number: PCT/US94/06727
(87) International publication number: WO 94/29282

(56) References cited:
- CHEMICAL ABSTRACTS, Volume 98, No. 14, issued 04 April 1983, (Columbus, Ohio), T. NIWA et al., "Anthraquinone Dyes for Cellulose-Containing Fibers", see the Abstract No. 108873Z; & DE,A,3 218 957, (16.12.1982).
- CHEMICAL ABSTRACTS, Vol. 101, No. 8, issued 20 August 1984, (Columbus, Ohio), SUMITOMO CHEMICAL CO. LTD, "Reactive Anthraquinone Dyes", see the Abstract No. 56488m; & JP,A,59 071 366, (23.04.1984).

## Description

This invention relates to the field of water-soluble fiber-reactive anthraquinone dyes.

The class of anthraquinone dyes is well known in the art. Anthraquinone dyes are generally considered to possess good dye properties. However, as a class they suffer from the disadvantages of high raw material cost. It is therefore important in the industrial dyeing and printing of substrates for a dye to provide superior dye properties and superior processing characteristics at a competitive cost.

U.S. Patent No. 5,112,971 represents an attempt to produce improved anthraquinone dyes having fiber reactive moieties useful in dyeing or printing substrates containing hydroxyl and/or amide groups. The dyes of this prior art reference may be represented by the following formula:

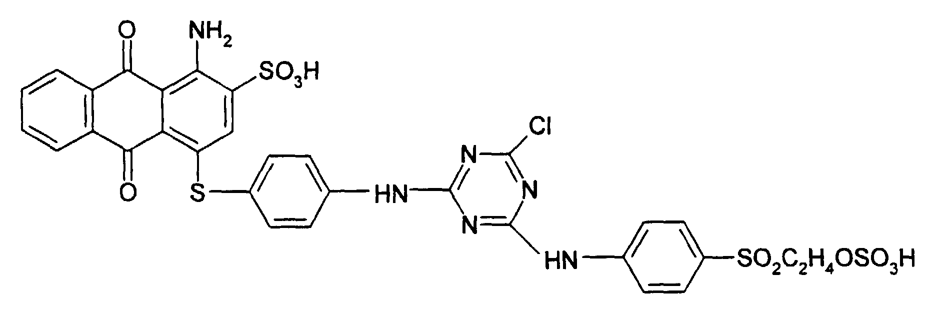

According to the present invention new fiber reactive dyes have been found corresponding to the general formula (1) 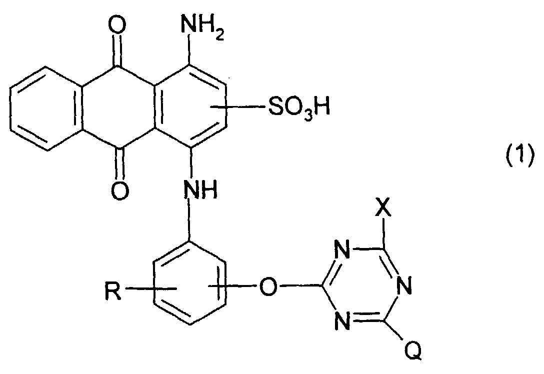 wherein
- R: is hydrogen or alkyl of 1 to 6 carbon atoms, such as methyl, ethyl, n-propyl and n-butyl, optionally substituted by hydroxy, methoxy, ethoxy, carboxy, sulfo, sulfato, cyano, chloro, bromo or fluoro, preferably by methoxy, ethoxy or sulfato, or is alkoxy of 1 to 6 carbon atoms optionally substituted by hydroxy, methoxy, ethoxy, carboxy, sulfo, sulfato, cyano, chloro, bromo or fluoro, preferably by methoxy, ethoxy or sulfato, or is sulfo or carboxy, and is preferably hydrogen;
- X: is fluoro, chloro, cyanoamino, hydroxy or a group of the formula (7a) 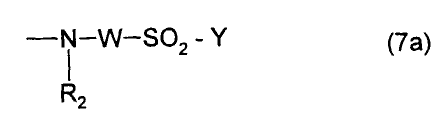 in which
W is a direct covalent bond, or is AR , ALK , AR-ALK or ALK-AR-ALK, wherein AR represents phenylene or naphthylene, each being optionally substituted by substituents selected from chloro, bromo, methyl, ethyl, methoxy, ethoxy, sulfo and carboxy, preferably by methyl, methoxy, sulfo and carboxy, and ALK represents alkylene of 1 to 6 carbon atoms which may be interrupted by a hetero atom selected from O , S and N , and
X is preferably fluoro, chloro, cyanoamino or hydroxy,
Y is vinyl or a group of the formula -CH₂-CH₂-Z wherein Z is a group capable of being split off by the action of an alkali reagent, selected from chloro, bromo, sulfato(-OSO₃H), thiosulfato(-S-SO₃H) and phosphato(-OPO₃H₂), and is most preferably sulfato, and Y is particularly preferable vinyl or sulfatoethyl, and
R₂ is hydrogen or alkyl of 1 to 6 carbon atoms, such as methyl, ethyl, n-propyl and n-butyl, which may be substituted by hydroxy, methoxy, ethoxy, carboxy, sulfo, sulfato, cyano, chloro, bromo or fluoro, preferably by methoxy, ethoxy or sulfato, or is a group of the formula -W-SO₂-Y wherein W and Y have one of the above-mentioned meanings, and is preferably hydrogen, methyl or ethyl;
- Q: is a group of the general formula (7a) in which W, Y and R₂ have one of the above-mentioned meanings.

The dyes of formula (1) provide dyeings and prints in brilliant blue shades having excellent fastness properties including chlorine, light and wet fastness, and they are easily synthesized. Another advantage is that the dyes of invention provide brilliant blue shades without metal complexing the dye, i.e. they are free of heavy metals. They may be applied by methods well known in the art for dyeing and printing textiles and other substrates.

Groups of the formula AR-ALK and ALK-AR-ALK are for example

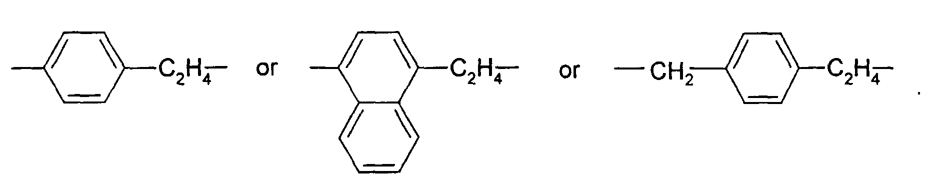

The anthraquinone dyes of the invention may be prepared using methods known in the art, such as described, for example, in U.S. Patent No. 5,112,971 which discloses a process for preparing anthraquinone dyes. Starting anthraquinone compounds of the general formula (2) 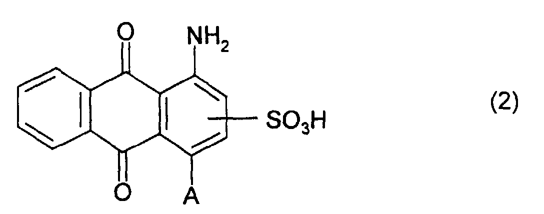 in which A is a leaving group such as halogen (Cl, F, Br, l), nitro, sulfo and the like, preferably halogen, are known as is their methods of preparation. They may be reacted according to Ullmann condensation reaction with a substituted or unsubstituted aminophenol of the formula (3) 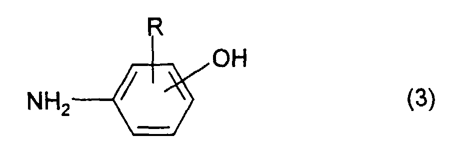 wherein R is defined above, to give the compound of the formula (4) 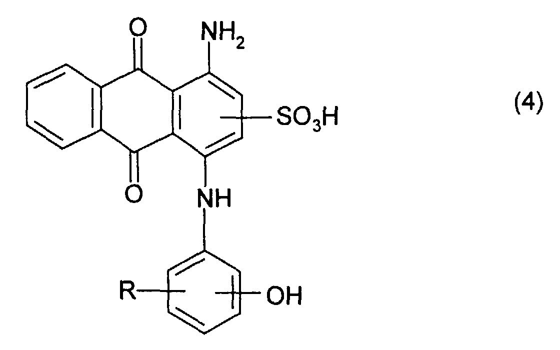 in which R is defined as above, which again is condensed with the condensation product of a chlorotriazine compound of the formula (5) 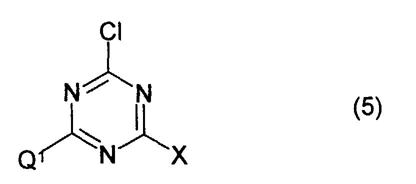 wherein Q¹ is halogen and X is defined above, to give a compound of the formula 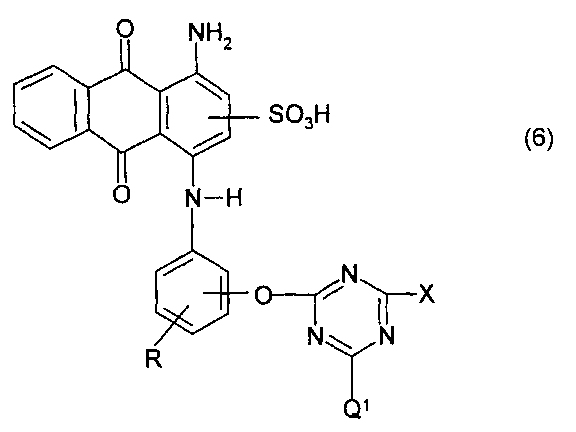 wherein R, X and Q¹ are defined as above, and replacing the halogen standing for Q¹ by the moiety of the above-mentioned formula (7a) by reacting this compound of formula (6) with an amine of the following general formula (7) 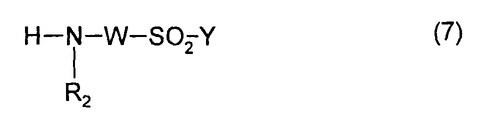 wherein R₂, W and Y are defined above.

It will be apparent to the skilled worker that the order of reaction may be modified, for example, that the amine of formula (7) may be reacted with the chlorotriazine compound of formula (5) and the resulting addition product may be condensed with the compound of formula (4). In addition, it would be obvious for the skilled worker to first condense the compound of formula (4) with chlorotriazine followed by the sequential addition of the moiety X and then the amine of formula (7). It will also be apparent to the skilled worker that the reaction mixture may be a mixture of fiber reactive moieties and depending on the reaction conditions, e.g. up to 30 % of the vinyl moiety and up to about 5 % of the non-reactive hydroxy moiety may be formed.

An anthraquinone of formula (2) is, for ex., 1-amino-4-bromoanthraquinone-2-sulfonic acid.

Exemplary aminophenols of formula (3) are:
4-aminophenol, 2-aminophenol, 3-aminophenol, 2-hydroxy-5-aminobenzenesulfonic acid, 2-amino-5-hydroxybenzenesulfonic acid, 2-hydroxy-4-aminobenzenesulfonic acid, 4-aminosalicyclic acid, 5-aminosalicyclic acid, 2-amino-4-methylphenol, 4-amino-2-methylphenol, 4-amino-3-methylphenol and 5-amino-2-methoxyphenol.

Groups of the formula radical Q are, for example, 2-(β-sulfatoethylsulfonyl)-phenylamino, 3-(β-sulfatoethylsulfonyl)-phenylamino, 4-(β-sulfatoethylsulfonyl)-phenylamino, 2-carboxy-5-(β-sulfatoethylsulfonyl)-phenylamino, 2-chloro-3-(β-sulfatoethylsulfonyl)-phenylamino, 2-chloro-4-(β-sulfatoethylsulfonyl)-phenylamino, 2-ethoxy-4- oder 5-(β-sulfatoethylsulfonyl)-phenyl-amino, 2-Ethyl-4-(β-sulfatoethylsulfonyl)-phenyl-amino, 2-Methoxy-5-(β-sulfatoethylsulfonyl)-phenyl-amino, 2,3-Dimethoxy-5-(β-sulfatoethylsulfonyl)-phenyl-amino, 2,4-Dimethoxy-5-(β-sulfatoethylsulfonyl)-phenyl-amino, 2,5-Dimethoxy-4-(β-sulfatoethylsulfonyl)-phenyl-amino, 2-Methoxy-5-methyl-4-(β-sulfatoethylsulfonyl)-phenyl-amino, 2- or 3- or 4-(β-Thiosulfatoethylsulfonyl)-phenyl-amino, 2-Methoxy-5-(β-thiosulfatoethylsulfonyl)-phenyl-amino, 2-Sulfo-4-(β-phosphatoethylsulfonyl)-phenyl-amino, 2-Sulfo-4-vinylsulfonyl-phenyl-amino, 2-Chloro-4- or -5-(β-chloroethylsulfonyl)-phenyl-amino, 5-(β-Sulfatoethylsulfonyl)naphth-2-yl-amino, 6- or 7- or 8-(β-Sulfatoethylsulfonyl)-naphth-2-yl-amino, 6-(β-Sulfatoethylsulfonyl)-1-sulfo-naphth-2-yl-amino, 5-(β-Sulfatoethylsulfonyl)-1-sulfo-naphth-2-yl-amino, 8-(β-Sulfatoethylsulfonyl)-6-sulfo-naphth-2-yl-amino, β-[4-(β'-Sulfatoethylsulfonyl)-phen]-ethylamino, β-[2-Sulfo-4-(β'-sulfatoethylsulfonyl)-phen]-ethylamino, β-(β'-Chloroethylsulfonyl)-ethylamino, β-(β'-Sulfatoethylsulfonyl)-ethylamino, β-(Vinylsulfonyl)-ethylamino, γ-(β'Chloroethylsulfonyl)-propylamino, γ-(β'-Sulfatoethylsulfonyl)-propylamino, γ-(β'-Bromoethylsulfonyl)- propylamino, γ-(Vinylsulfonyl)-propylamino, 1-Methyl-1-(β-sulfatoethylsulfonyl)-1-ethylamino, δ-(β'-Sulfatoethylsulfonyl)-butylamino, 2-Methyl-2-(β-chloroethylsulfonyl)-1-propylamino, $\overline{\text{ω}}$-(β'-Chloroethylsulfonyl)-pentylamino, β-(β'Chloroethylsulfonyl)-n-hexylamino, N-Methyl-N-[β-(β'chloroethylsulfonyl)-ethyl]-amino, N-Ethyl-N-[β-(β'-chloroethylsulfonyl)-ethyl]-amino, N-n-Propyl-N-[β-(β'chloroethylsulfonyl)-ethyl]-amino, N-Carboxymethyl-N-[β-(β'-bromoethylsulfonyl)-ethyl]-amino, N-Sulfatomethyl-N-[β-(β'-chloroethylsulfonyl)-ethyl]-amino, N-(β-Carboxyethyl)-N-[γ'-(β-chloroethylsulfonyl)-propyl]-amino- N-(β-Sulfatoethyl)-N-[γ'-(β''-chloroethylsulfonyl)-propyl]-amino, N-(β-Sulfatoethyl)-N-[δ'-(β''chloroethylsulfonyl)-butyl]-amino, N-(β-Ethoxyethyl)-N-[δ'-(β"-chloroethylsulfonyl)-butyl]-amino, N-(γ-Chloropropyl)-N-[β'-(β"-chloroethylsulfonyl)-ethyl]-amino,
Bis-[β-(β'-chloroethylsulfonyl)-ethyl]-amino,
Bis-[β-(β'-bromoethylsulfonyl)-ethyl]-amino,
Bis-[γ-(β'-chloroethylsulfonyl)-propyl]-amino,
Bis-[δ-(β'-chloroethylsulfonyl)-butyl]-amino,
Bis-(β-vinylsulfonyl-ethyl)-amino, N-(β-Cyanoethyl)-
N-[γ'-(β''-chloroethylsulfonyl)-propyl]-amino,
β-[β'-(β''-Chloroethylsulfonyl)-ethylamino]-ethylamino,
β-[β'(β''-Sulfatoethylsulfonyl)-ethylamino]-ethylamino,
β-[β'(β''-Chloroethylsulfonyl)-ethoxy]-ethylamino,
β-[β'-(β"-Sulfatoethylsulfonyl)-ethoxy]-ethylamino,
N-Ethyl-[4-(β-sulfato-ethylsulfonyl) phenyl]-amino,
N-Methyl-[3-(β-sulfato-ethylsulfonyl) phenyl]-amino,
4-[γ-(β'Sulfatoethylsulfonyl)-propoxy]-phenylamino,
N-Methyl-N-[4-(β-sulfatoethylsulfonyl)-phenyl]-amino,
N-Methyl-N-[3-(β-sulfatoethylsulfonyl)-phenyl]-amino,
N-Ethyl-N-(4-(β-sulfatoethylsulfonyl)-phenyl]-amino,
N-Ethyl-N-[3-(β-sulfatoethylsulfonyl)-phenyl]-amino.

The amino moieties (Q) are obtained from the corresponding amines e.g.:
2,5-Disulfo-aniline;
2-(β-Sulfatoethylsulfonyl)-aniline;
3-(β-Sulfatoethylsulfonyl)-aniline;
4-(β-Sulfatoethylsulfonyl)-aniline;
2-Carboxy-5-(β-sulfatoethylsulfonyl)-aniline;
2-Chloro-3-(β-Sulfatoethylsulfonyl)-aniline;
2-Chloro-4-(β-sulfatoethylsulfonyl)-aniline;
2-Ethoxy-4- or 5-(β-sulfatoethylsulfonyl)-aniline;
2-Ethyl-4-(β-sulfatoethylsulfonyl)-aniline;
2-Methoxy-5-(β-sulfatoethylsulfonyl)-aniline;
2,3-Dimethoxy-5-(β-sulfatoethylsulfonyl)-aniline;
2,4-Dimethoxy-5-(β-sulfatoethylsulfonyl)-aniline;
2,5-Dimethoxy-4-(β-sulfatoethylsulfonyl)-aniline;
2-Methoxy-5-methyl-4-(β-sulfatoethylsulfonyl)-aniline;
2- or 3- or 4-(β-thiosulfatoethylsulfonyl)-aniline;
2-Methoxy-5-(β-thiosulfatoethylsulfonyl)-aniline;
2-Sulfo-4-(β-phosphatoethylsulfonyl)-aniline;
2-Sulfo-4-vinylsulfonyl-aniline;
2-Chloro-4- or -5-(β-chloroethylsulfonyl)aniline;
N-Methyl-N-[4-(β-sulfatoethylsulfonyl)] aniline;
N-Methyl-N-[3-(β-sulfatoethylsulfonyl)] aniline;
N-Ethyl-N-[4-(β-sulfatoethylsulfonyl)] aniline;
N-Ethyl-N-[3-(β-sulfatoethylsulfonyl)] aniline.

After preparation, the dyes may be isolated as a powder, either by salting out of solution or by spray drying, and brought to standard strength by the addition of inorganic salt, generally sodium sulfate. Advantageously, the prepared dyestuff may be used directly as a liquid composition after standardizing with water. Such liquid compositions will contain from 5 to 45% (by weight) of the dyes of the invention.

The dyes of the invention in this description are shown in their free acid form. They may be employed in their free acid form or as salts of the acid. Preferably, they are used in their salt form and in particular as the alkali metal and alkaline earth metal salts; e.g. as sodium, potassium, lithium and the like.

The dyes of the invention may be employed to dye materials such as cotton, linen, viscose, rayon, wool, silk and synthetic polyamides by methods well known in the art e.g. exhaust dyeing methods such beck, jet, and package dyeing or continuous pad dyeing techniques.

The following examples illustrate the invention but are not intended to limit its scope unless otherwise specified. In the examples and the claims, percentages and parts are by weight and temperatures are °C.

### Example 1

A dye of the following formula was made:

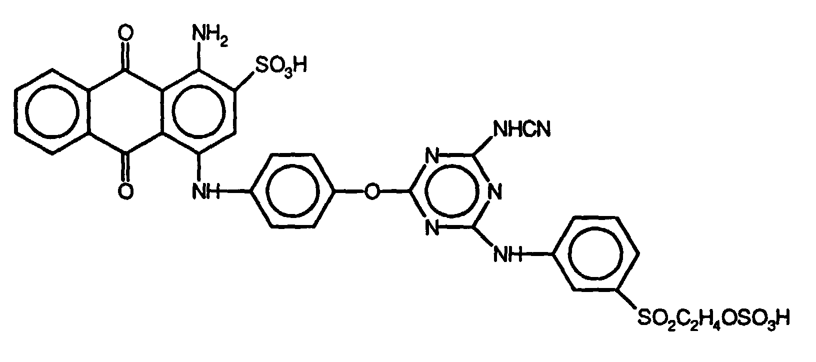

1.3 parts of cyanamide and 5.6 parts of cyanuric chloride were condensed at 0-5°C in an aqueous medium of pH 8.5-9.5. The product was condensed with 15.3 parts of 1-amino-4-[(4-hydroxyanilino)]-9,10-dioxo-2-anthracenesulfonic acid at 10-30°C and at a pH of 8-10. This second product was condensed with 8.5 parts of 1-aminobenzene-3-(2-sulfatoethyl)sulfone at 40-50°C and at a pH of 3.5-4.0. The product was isolated by salting out with 20 parts of sodium chloride followed by filtration to afford 24 parts of a greenish blue dye having an absorbance of 0.350 at a lambda max of 603 nm and an assay of 79% by HPLC.

### Example 2:

A dye of the following formula was made:

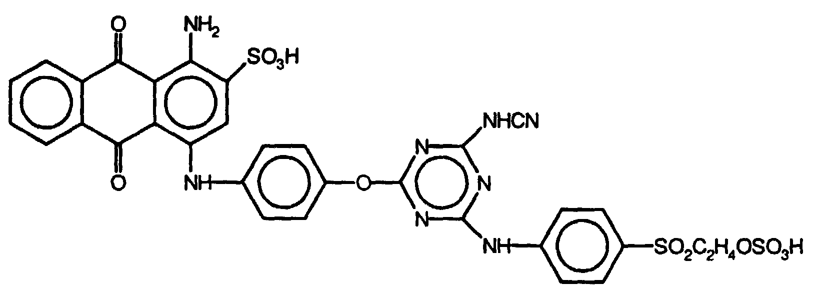

This compound was prepared by the above procedure substituting 1-aminobenzene-4-(2-sulfatoethyl)sulfone for 1-aminobenzene-3-(2-sulfatoethyl)sulfone. The product was a greenish blue dye having an absorbance of 0.300 at a lambda max of 603 nm and an assay of 70% by HPLC.

Similar dyes may be prepared using the foregoing procedures. These dyes have the following general formula and are illustrated below: 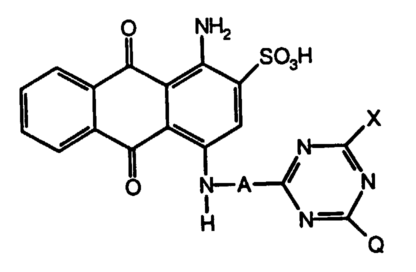 wherein: X is F, Cl, NHCN or OH.

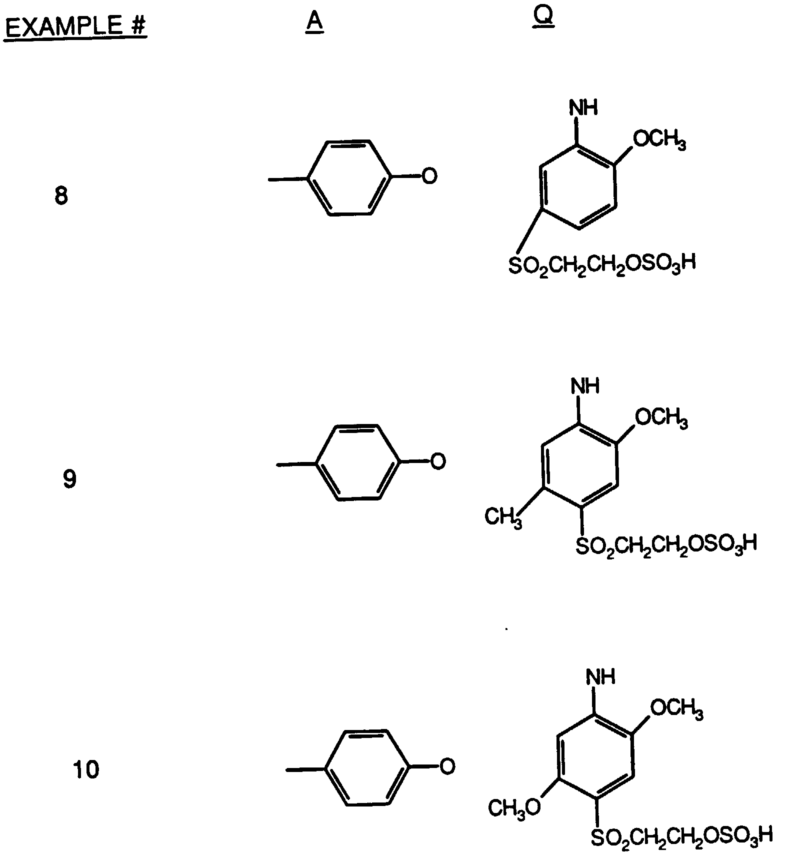

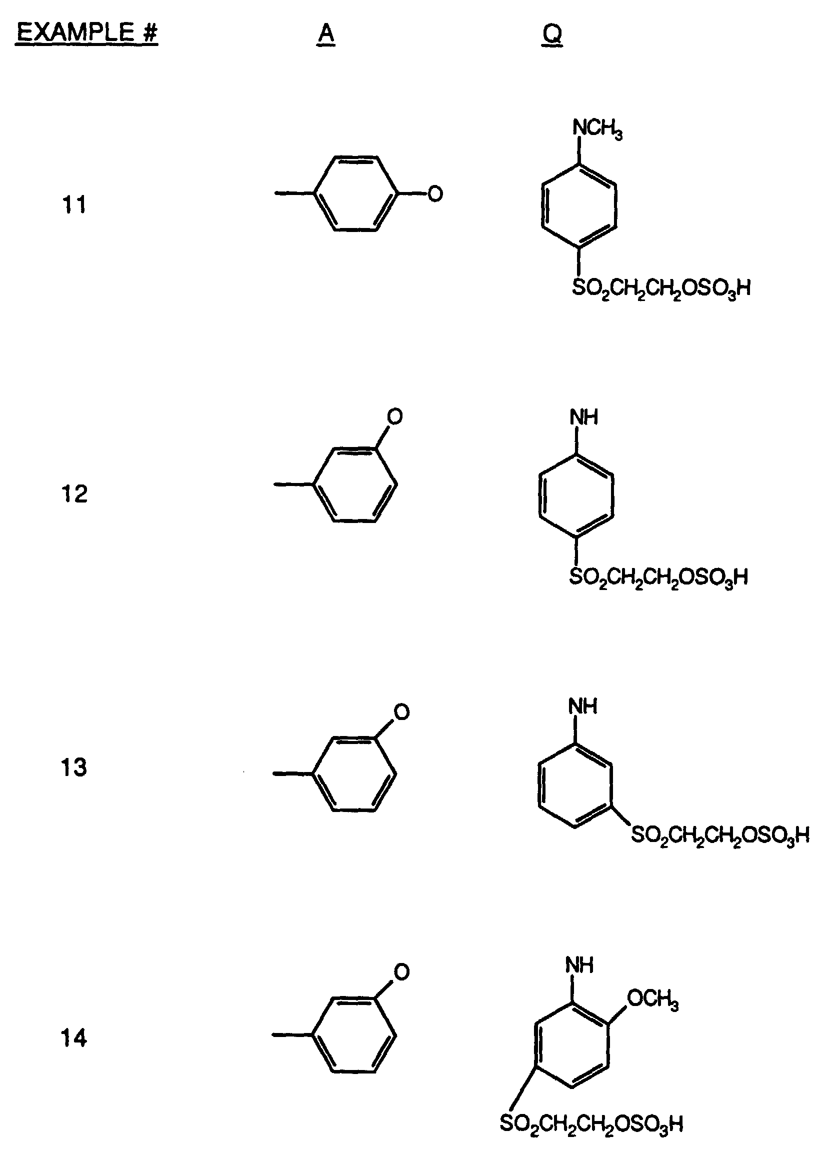

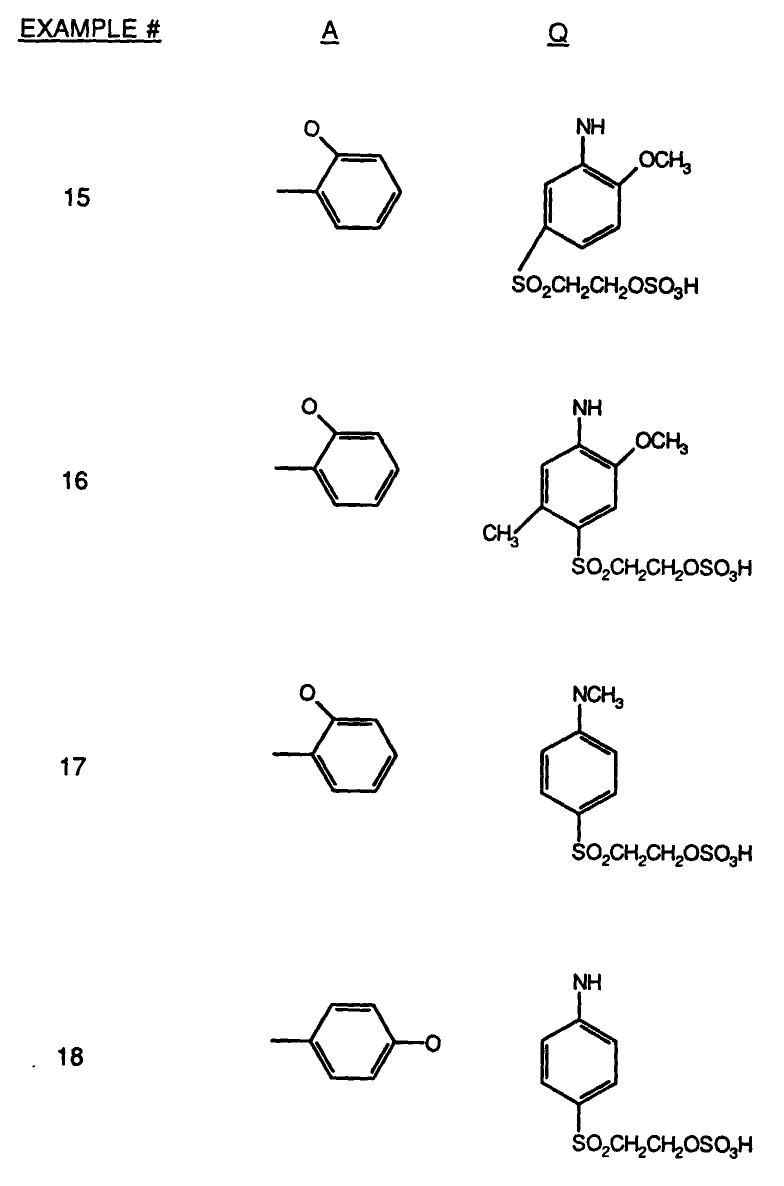

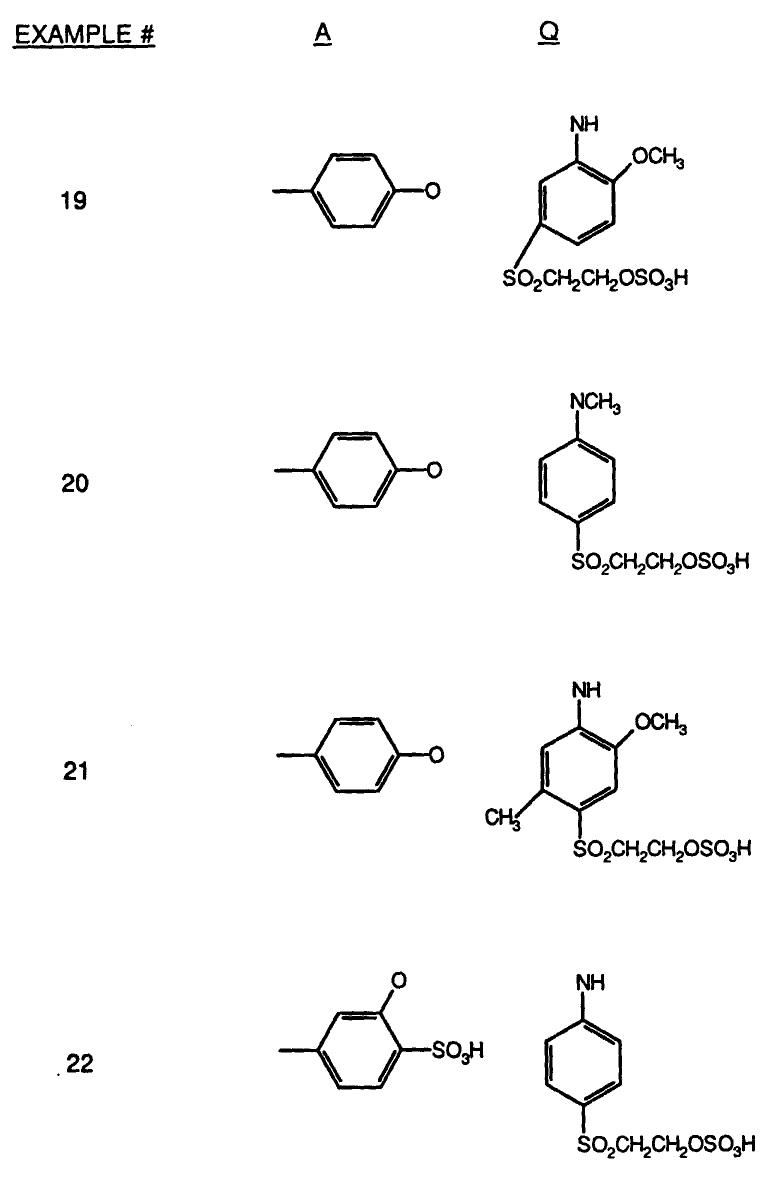

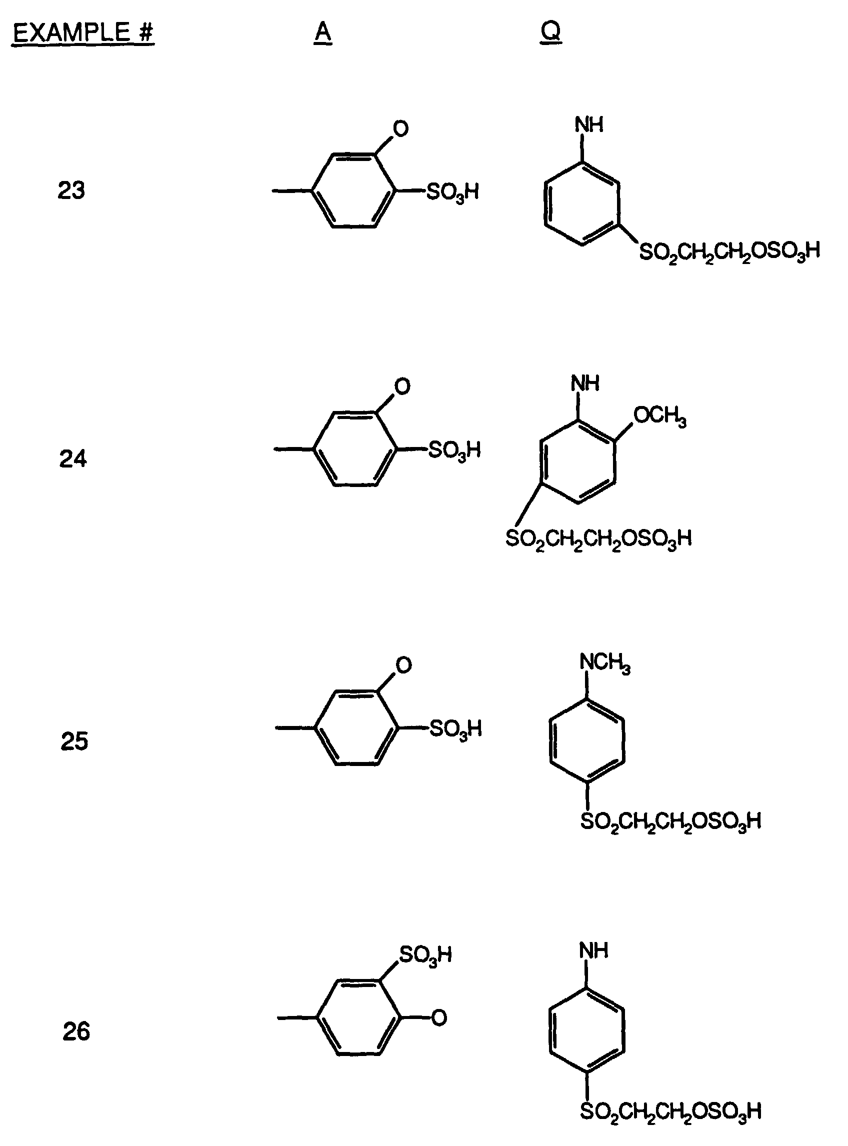

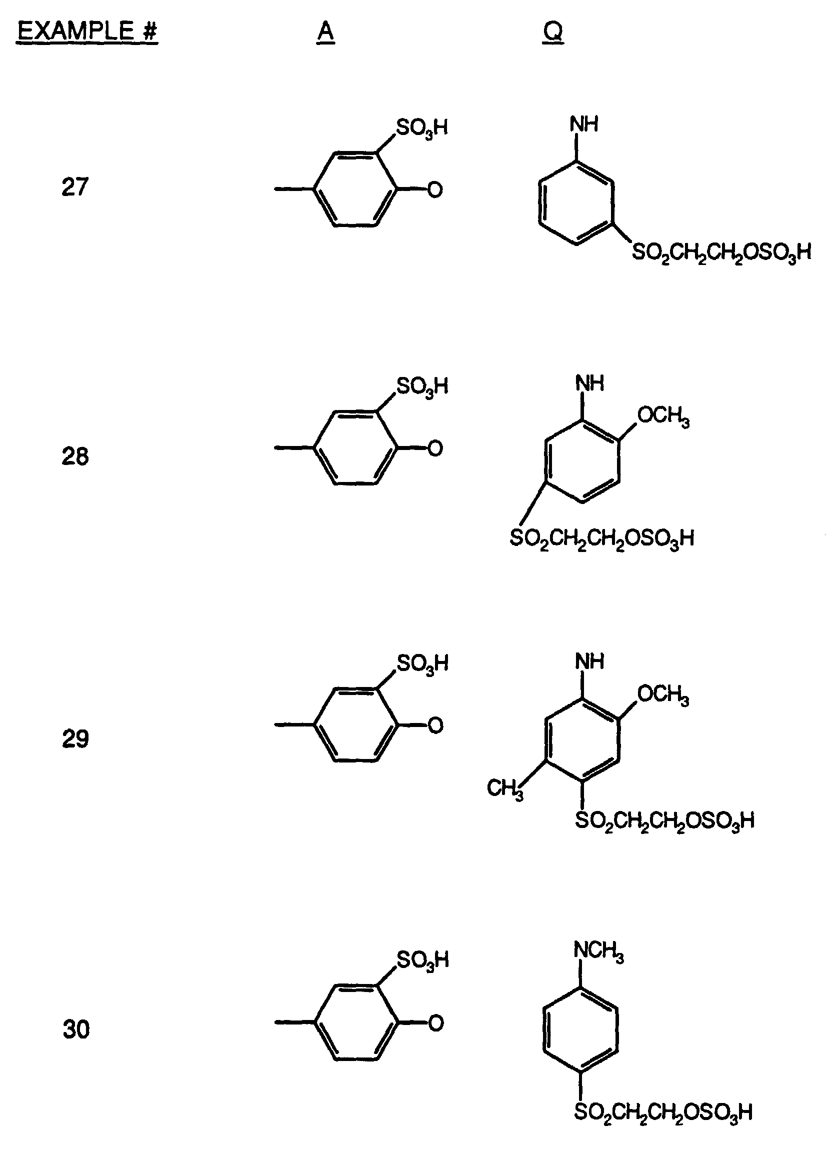

### Example 31

Example 8 may be repeated wherein the moiety Q is a group of the formula

-NH-CH₂-CH₂-CH₂-SO₂-CH = CH₂.

### Example 32

Example 8 may be repeated wherein the moiety Q is a group of the formula

-N(CH₂-CH₂-SO₂-CH = CH₂)₂.

### Example 33

Example 8 may be repeated wherein the moiety Q is a group of the formula

-NH-CH₂-CH₂-O-CH₂-CH₂-SO₂-CH = CH₂.

### Example 34

Example 8 may be repeated wherein the moiety Q is a group of the formula

-(CH₂-CH₂-CH₂-SO₂-CH₂-CH₂-Cl)₂.

### Example 35

Example 8 may be repeated wherein the moiety Q is a group of the formula

-N(CH₂-CH₂-CH₂-SO₂-CH = CH₂)₂.

Similarly, dyes may be prepared using the foregoing procedures. These dyes have the following general formula and are illustrated below: 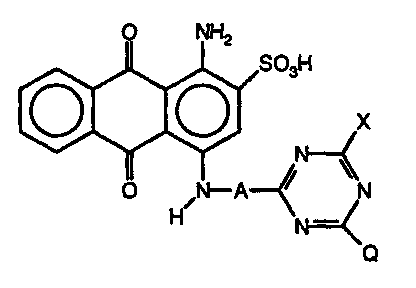 wherein: X is Q.

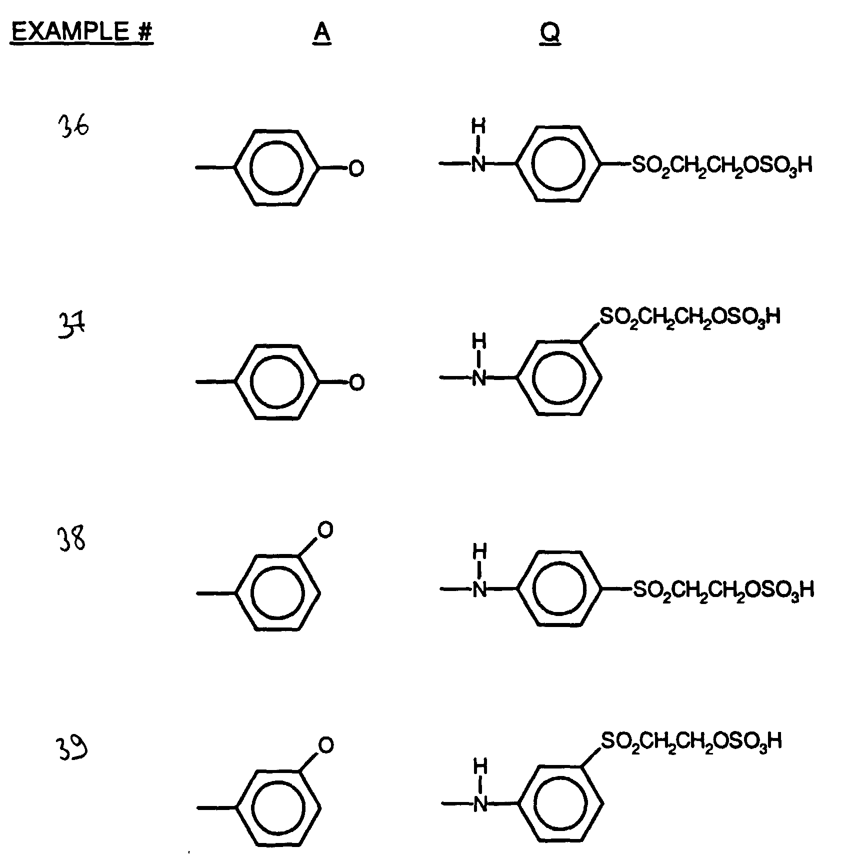

## Claims

1. A compound of the formula (1) 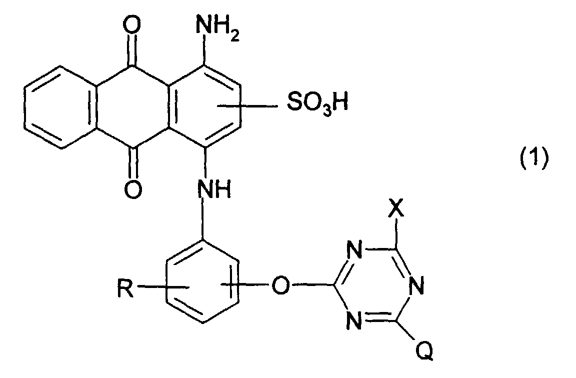 wherein
R is hydrogen or alkyl of 1 to 6 carbon atoms optionally substituted by hydroxy, methoxy, ethoxy, carboxy, sulfo, sulfato, cyano, chloro, bromo or fluoro, or is alkoxy of 1 to 6 carbon atoms optionally substituted by hydroxy, methoxy, ethoxy, carboxy, sulfo, sulfato, cyano, chloro, bromo or fluoro, or is sulfo or carboxy;
X is fluoro, chloro, cyanoamino, hydroxy or a group of the formula (7a) 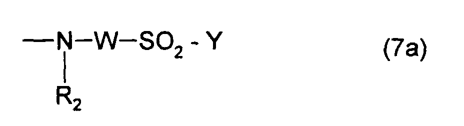 in which
W is a direct covalent bond, or is AR , ALK , AR-ALK or ALK-AR-ALK, wherein AR represents phenylene or naphthylene, each being optionally substituted by substituents selected from chloro, bromo, methyl, ethyl, methoxy, ethoxy, sulfo and carboxy, and ALK represents alkylene of 1 to 6 carbon atoms which may be interrupted by a hetero atom selected from O , S and N ,
Y is vinyl or a group of the formula -CH₂-CH₂-Z wherein Z is a group capable of being split off by the action of an alkali reagent selected from chloro, bromo, sulfato, thiosulfato and phosphato, and
R₂ is hydrogen or alkyl of 1 to 6 carbon atoms, which may be substituted by hydroxy, methoxy, ethoxy, carboxy, sulfo, sulfato, cyano, chloro, bromo or fluoro, or is a group of the formula -W-SO₂-Y wherein W and Y have one of the above-mentioned meanings;
Q is a group of the general formula (7a) in which W, Y and R₂ have one of the above-mentioned meanings.

2. A compound according to claim 1, wherein W is alkylene of 1 to 6 carbon atoms which may be interrupted by a hetero atom selected from O , S and N .

3. A compound according to claim 1, wherein X is fluoro or chloro or is cyanoamino or hydroxy, and Z is chloro, bromo, sulfato or phosphato.

4. A compound according to claim 1, wherein Z is sulfato.

5. A compound according to claim 3 of the formula

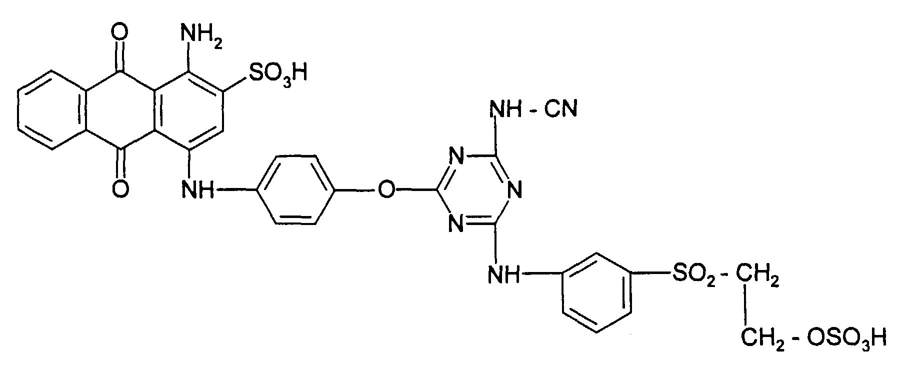

6. A compound according to Claim 3 of the following formula:

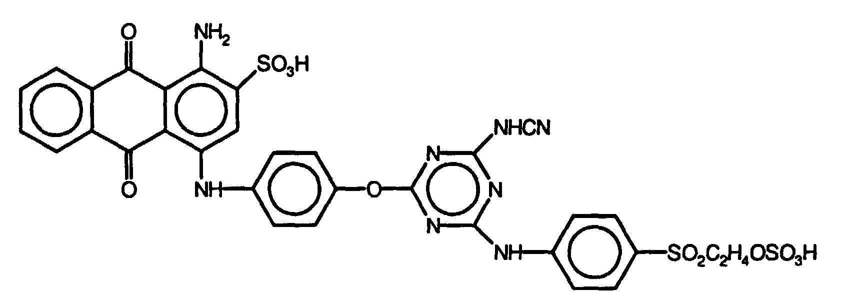

7. A compound according to Claim 3 of the following formula:

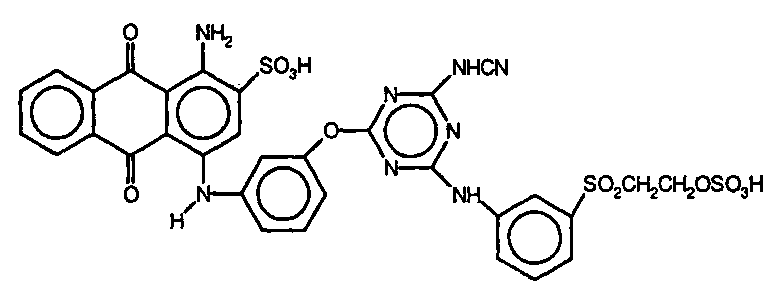

8. A compound according to Claim 3 of the following formula:

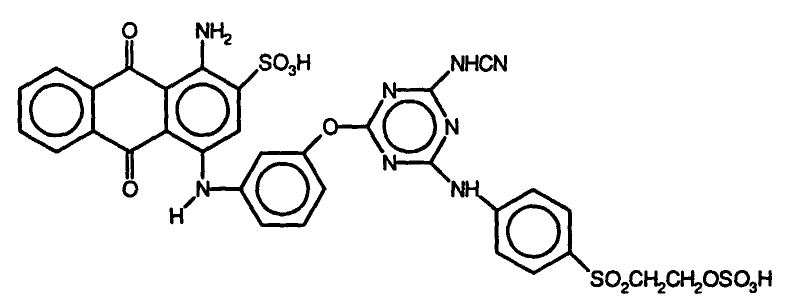

## Patentansprüche

1. Verbindung der Formel (1) 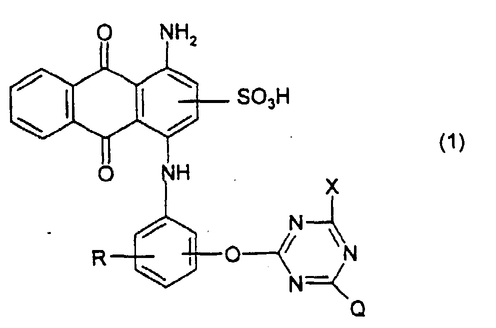 worin
R für Wasserstoff, für gegebenenfalls durch Hydroxy, Methoxy, Ethoxy, Carboxy, Sulfo, Sulfato, Cyano, Chlor, Brom oder Fluor substituiertes Alkyl mit 1 bis 6 C-Atomen, für gegebenenfalls durch Hydroxy, Methoxy, Ethoxy, Carboxy, Sulfo, Sulfato, Cyano, Chlor, Brom oder Fluor substituiertes Alkoxy mit 1 bis 6 C-Atom oder für Sulfo oder Carboxy steht;
X Fluor, Chlor, Cyanoamino, Hydroxy oder eine Gruppe der Formel (7a) bedeutet, 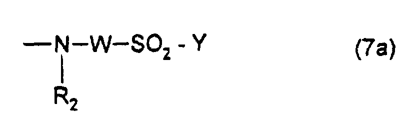 in der
W eine direkte kovalente Bindung oder AR, ALK, AR-ALK oder ALK-AR-ALK bezeichnet, wobei AR für Phenylen oder Naphthylen, das jeweils gegebenenfalls durch Substituenten aus der Reihe Chlor, Brom, Methyl, Ethyl, Methoxy, Ethoxy, Sulfo und Carboxy substituiert ist, und ALK für gegebenenfalls durch ein Heteroatom aus der Reihe O, S und N unterbrochenes Alkylen mit 1 bis 6 C-Atomen steht,
Y Vinyl oder eine Gruppe der Formel -CH₂-CH₂-Z ist, in welcher Z einen alkalisch eliminierbaren Rest aus der Reihe Chlor, Brom, Sulfato, Thiosulfato und Phosphato bedeutet, und
R₂ Wasserstoff, gegebenenfalls durch Hydroxy, Methoxy, Ethoxy, Carboxy, Sulfo, Sulfato, Cyano, Chlor, Brom oder Fluor substituiertes Alkyl mit 1 bis 6 C-Atomen oder eine Gruppe der Formel -W-SO₂-Y bedeutet, wobei W und Y eine der obengenannten Bedeutungen besitzen;
Q für eine Gruppe der allgemeinen Formel (7a) steht, in der W, Y und R₂ eine der obengenannten Bedeutungen besitzen.

2. Verbindung nach Anspruch 1, bei der W für gegebenenfalls durch ein Heteroatom aus der Reihe O, S und N unterbrochenes Alkylen mit 1 bis 6 C-Atomen steht.

3. Verbindung nach Anspruch 1, bei der X für Fluor oder Chlor oder für Cyanoamino oder Hydroxy und Z für Chlor, Brom, Sulfato oder Phosphato steht.

4. Verbindung nach Anspruch 1, bei der Z für Sulfato steht.

5. Verbindung nach Anspruch 3 der Formel

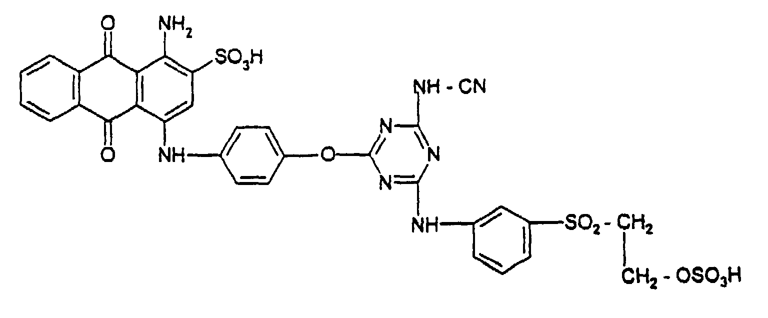

6. Verbindung nach Anspruch 3 der nachstehenden Formel:

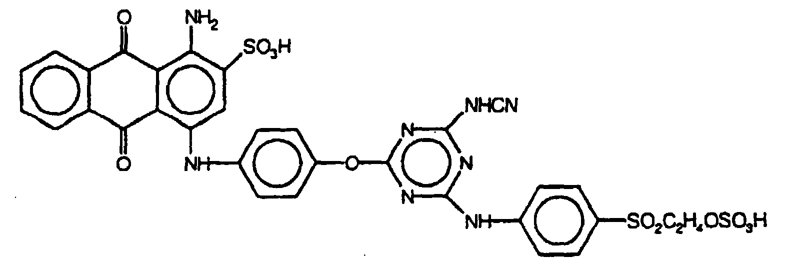

7. Verbindung nach Anspruch 3 der nachstehenden Formel:

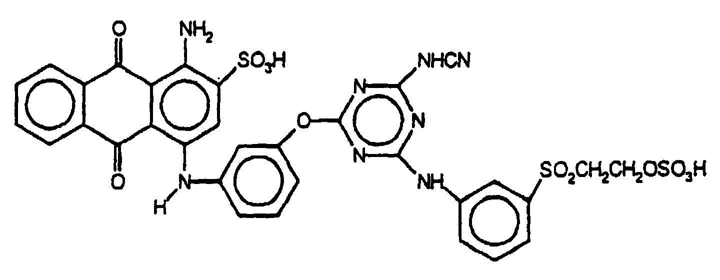

8. Verbindung nach Anspruch 3 der nachstehenden Formel:

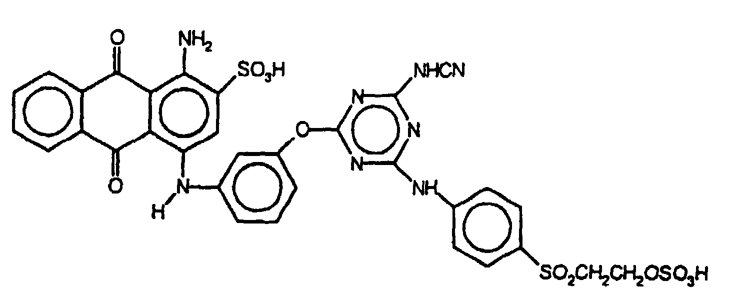

## Revendications

1. Composé de formule (1) 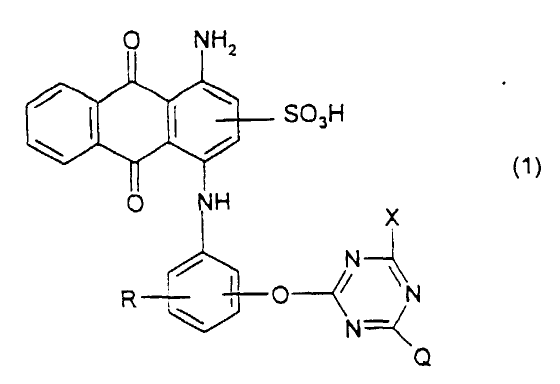 dans laquelle
R est un hydrogène ou un alkyle de 1 à 6 atomes de carbone facultativement substitué par hydroxy, méthoxy, éthoxy, carboxy, sulfo, sulfato, cyano, chlore, brome ou fluor, ou est un alcoxy de 1 à 6 atomes de carbone facultativement substitué par hydroxy, méthoxy, éthoxy, carboxy, sulfo, sulfato, cyano, chlore, brome ou fluor, ou est un sulfo ou un carboxy;
X est un fluor, un chlore, un cyanoamino, un hydroxy ou un groupement de formule (7a) 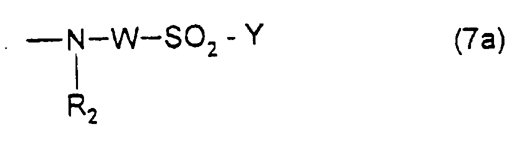 dans laquelle
W est une liaison covalente directe, ou est AR, ALK, AR-ALK ou ALK-AR-ALK, dans lesquels AR représente un phénylène ou un naphtylène, chacun étant facultativement substitué par des substituants sélectionnés parmi chlore, brome, méthyle, éthyle, méthoxy, éthoxy, sulfo et carboxy, et ALK représente un alkylène de 1 à 6 atomes de carbone qui peuvent être interrompus par un hétéroatome sélectionné parmi O, S et N,
Y est un vinyle ou un groupement de formule -CH₂-CH₂-Z, dans laquelle Z est un groupement pouvant être clivé par l'action d'un réactif alcalin sélectionné parmi un chlore, un brome, un sulfato, un thiosulfato et un phosphato, et
R₂ est un hydrogène ou un alkyle de 1 à 6 atomes de carbone qui peut être substitué par hydroxy, méthoxy, éthoxy, carboxy, sulfo, sulfato, cyano, chlore, brome ou fluor, ou est un groupement de formule -W-SO₂-Y dans laquelle W et Y ont une des significations susmentionnées;
Q est un groupement de formule générale (7a) dans laquelle W, Y et R₂ ont une des significations susmentionnées.

2. Composé suivant la revendication 1, dans lequel W est un alkylène de 1 à 6 atomes de carbone qui peut être interrompu par un hétéroatome sélectionné parmi O, S et N.

3. Composé suivant la revendication 1, dans lequel X est un fluor ou un chlore ou est un cyanoamino ou un hydroxy, et Z est un chlore, un brome, un sulfato ou un phosphato.

4. Composé suivant la revendication 1, dans lequel Z est un sulfato.

5. Compasé suivant la revendication 3, de la formule,

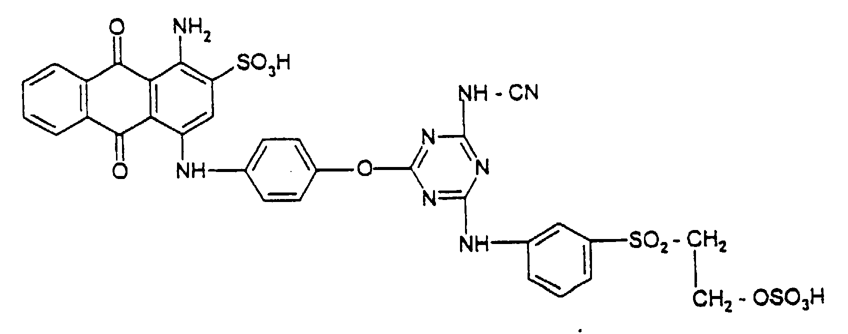

6. Composé suivant la revendication 3, de la formule suivante :

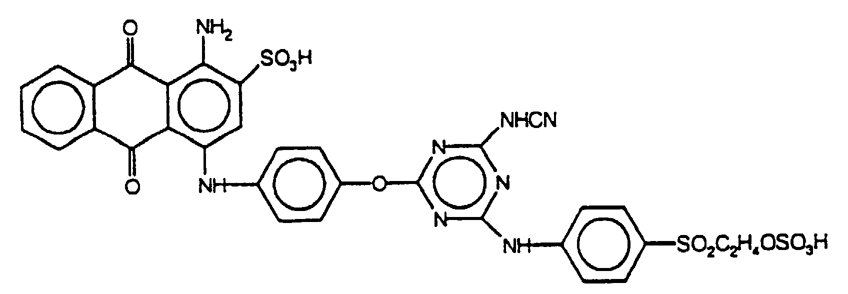

7. Compasé suivant la revendication 3, de la formule suivante :

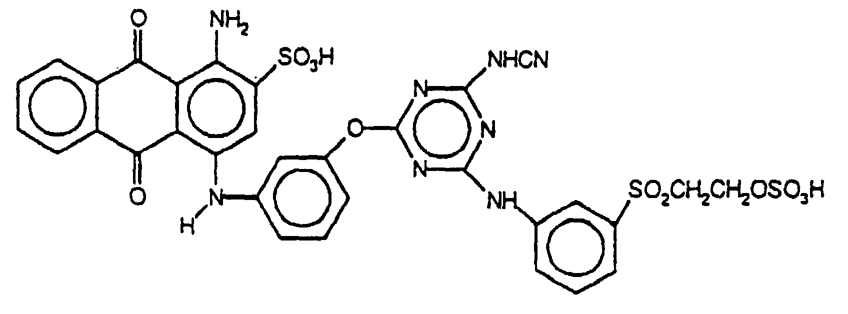

8. Composé suivant la revendication 3, de la formule suivante :
